(19) Europäisches Patentamt
European Patent Office
Office européen des brevets

(11) **EP 3 559 083 B1**

(12) **EUROPEAN PATENT SPECIFICATION**

(45) Date of publication and mention
of the grant of the patent:
**18.11.2020   Bulletin 2020/47**

(51) Int Cl.:
***C08G 65/30*** *(2006.01)*          ***C07C 41/34*** *(2006.01)*
***C02F 1/42*** *(2006.01)*

(21) Application number: **17822909.2**

(22) Date of filing: **11.12.2017**

(86) International application number:
**PCT/US2017/065537**

(87) International publication number:
**WO 2018/118474 (28.06.2018 Gazette 2018/26)**

(54) **PROCESSES FOR PURIFYING POLYETHER POLYOLS**

VERFAHREN ZUR REINIGUNG VON POLYETHERPOLYOLEN

PROCÉDÉS POUR LA PURIFICATION DE POLYOLS DE POLYÉTHER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.12.2016   US 201662437190 P**

(43) Date of publication of application:
**30.10.2019   Bulletin 2019/44**

(73) Proprietor: **Covestro LLC
Pittsburgh, PA 15205 (US)**

(72) Inventors:
• **WOOD, William E.**
**Hurricane, West Virginia 25526 (US)**
• **CALES, Jay**
**Hurricane, West Virginia 25526 (US)**

• **RODBERG, Steven J.**
**Charleston, West Virginia 25304 (US)**
• **SMITH, Joanne**
**Scott Depot, West Virginia 25560 (US)**
• **REESE, Jack R.**
**Coraopolis, Pennsylvania 15108 (US)**
• **MORRISON, D. Mark**
**Moon Township, Pennsylvania 15108 (US)**

(74) Representative: **Levpat
c/o Covestro AG
Gebäude 4825
51365 Leverkusen (DE)**

(56) References cited:
**EP-A2- 0 376 157          US-A- 4 994 627
US-A1- 2003 150 814**

## Description

## FIELD

The present invention is directed to, among other things, processes for purifying polyether polyols via treatment with a cation exchange resin.

## BACKGROUND

Polyether polyols are often manufactured using a catalyzed reaction of initiators having active hydrogen atoms with epoxides such as, for example, ethylene oxide and/or propylene oxide. Alkalinity is introduced into the polyether polyols, for example, by using alkaline metal hydroxides as catalysts.

Potassium hydroxide (KOH) and sodium hydroxide (NaOH) are some examples of typical alkaline catalysts used. In general, the metal hydroxide catalyst is added to the starter (usually a hydroxyl group containing compound), and equilibrium between the metal hydroxide and the starter occurs. This equilibrium is as shown in the following equation (using KOH as the alkaline catalyst):

$$KOH + ROH \leftrightarrow H_2O + RO^-K^+$$

Both the hydroxide and the alkoxide can react with epoxides. This is often acceptable for short chain (low molecular weight) polyols, but the reaction of water is undesirable in the preparation of long chain (i.e., high molecular weight) polyols. It is therefore, necessary to force the above equilibrium to the right by removing the water (i.e., dewatering). This converts hydroxide to alkoxide. The total amount of alkalinity remains constant and is equal to the amount of KOH originally added.

Once the polymerization of the epoxide(s) is completed, the resulting crude, polyether polyol contains alkaline ions from the catalyst that must be removed until a very low level of such alkaline ions (often 5-10 ppm) remains. Several processes for the removal of such alkaline ions are known.

One efficient method of removing alkaline ions from a crude polyether polyols is by treatment with an acidic cation exchange resin. In this process, the crude polyether polyol is passed through a porous bed comprising the cation exchange resin, which is sometimes a copolymer of styrene and divinylbenzene with sulfonic acid groups, whereby an ion exchange occurs between the alkaline ions in the crude polyether polyol and the cation exchange sites on the resin, thereby purifying the polyether polyol.

Such ion exchange purification processes are, however, not without their disadvantages. Notably, the cation exchange resin needs to be regenerated periodically by treatment with an acid solution. Such regeneration produces significant wastewater that must be treated and can also impact manufacturing productivity since polyether polyols cannot be purified while such regeneration process is ongoing (unless, for example, a second, costly, purification installation is present that can be used when the first installation is undergoing regeneration). Because of such disadvantages, the use of ion exchange purification processes is not widely used industrially for the purification of polyether polyols.

US 2003/0150814 A1 disclose a method for separating alkali metal ions from alkoxylates containing metal ions, wherein the alkali metal ions are separated off from alkoxylates containing alkali metal ions by a process comprising: a) dilution of the alkali metal-containing alkoxylate with an inert solvent, b) treatment of the alkali metal-containing solution of the alkoxylate with a cationic exchanger in order to obtain a substantially alkali metal-free solution of the alkoxylate, and c) removal of the solvent from the substantially alkali metal-free solution of the alkoxylate in order to obtain a substantially alkali metal-free and substantially solvent-free alkoxylate.

In EP 0 376 157 A2 a process for purification of catalysts from polyols using ion exchange resins is disclosed, wherein said catalyst is an alkali hydroxide or alkoxide comprising the sequential steps of: a) mixing the polyether molecule having a molecular weight of 500-10,000 with water, b). blending into the product of (a) a sufficient amount of a lower aliphatic alcohol to break the emulsion formed in step (a), c) passing the product of step (b) through a macroporous cation exchange resin to obtain a purified polyether molecule, methanol, and water.

US 4 994 627 (A) describe the purification of polyoxyalkylene glycols wherein the alkaline crude glycol feedstock is contacted with a basic ion exchange resin in the presence of water at defined temperature, pressure and order to reduce the impurity level of alkaline ions to less than 100 ppm.

As a result, it would be desirable to provide an ion exchange polyether polyol purification process that has improved alkaline ion removal efficiency, thereby reducing frequency in which cation exchange resin regeneration needs to be performed.

The present invention was made in view of the foregoing desire.

## SUMMARY OF THE INVENTION

In certain respects, the present specification is directed to processes for removing alkali metal ions from a polyether polyol. These processes of the present specification comprise: (a) combining a mixture comprising water and a polar organic solvent with a mixture comprising a polyether polyol and an alkali metal ion-containing catalyst; and (b) passing the product of step (a) through a bed comprising a cation exchange resin that is disposed in a container to remove alkali metal ions therefrom. Moreover, in these processes, the container is operated liquid-full throughout the process.

The present specification is also directed to, among other things, systems for conducting such processes, polyether polyols purified by such processes, and polyurethanes, such as polyurethane foams, produced from such polyether polyols.

## DETAILED DESCRIPTION

Various embodiments are described and illustrated in this specification to provide an overall understanding of the structure, function, properties, and use of the disclosed inventions. It is understood that the various embodiments described and illustrated in this specification are non-limiting and non-exhaustive. Thus, the invention is not limited by the description of the various non-limiting and non-exhaustive embodiments disclosed in this specification. The features and characteristics described in connection with various embodiments may be combined with the features and characteristics of other embodiments. Such modifications and variations are intended to be included within the scope of this specification. As such, the claims may be amended to recite any features or characteristics expressly or inherently described in, or otherwise expressly or inherently supported by, this specification. Further, Applicant(s) reserve the right to amend the claims to affirmatively disclaim features or characteristics that may be present in the prior art. Therefore, any such amendments comply with the requirements of 35 U.S.C. § 112 and 35 U.S.C. § 132(a). The various embodiments disclosed and described in this specification can comprise, consist of, or consist essentially of the features and characteristics as variously described herein.

Any patent, publication, or other disclosure material identified herein is incorporated by reference into this specification in its entirety unless otherwise indicated, but only to the extent that the incorporated material does not conflict with existing definitions, statements, or other disclosure material expressly set forth in this specification. As such, and to the extent necessary, the express disclosure as set forth in this specification supersedes any conflicting material incorporated by reference herein. Any material, or portion thereof, that is said to be incorporated by reference into this specification, but which conflicts with existing definitions, statements, or other disclosure material set forth herein, is only incorporated to the extent that no conflict arises between that incorporated material and the existing disclosure material. Applicant(s) reserves the right to amend this specification to expressly recite any subject matter, or portion thereof, incorporated by reference herein.

In this specification, other than where otherwise indicated, all numerical parameters are to be understood as being prefaced and modified in all instances by the term "about", in which the numerical parameters possess the inherent variability characteristic of the underlying measurement techniques used to determine the numerical value of the parameter. At the very least, and not as an attempt to limit the application of the doctrine of equivalents to the scope of the claims, each numerical parameter described in the present description should at least be construed in light of the number of reported significant digits and by applying ordinary rounding techniques.

Also, any numerical range recited in this specification is intended to include all sub-ranges of the same numerical precision subsumed within the recited range. For example, a range of "1.0 to 10.0" is intended to include all sub-ranges between (and including) the recited minimum value of 1.0 and the recited maximum value of 10.0, that is, having a minimum value equal to or greater than 1.0 and a maximum value equal to or less than 10.0, such as, for example, 2.4 to 7.6. Any maximum numerical limitation recited in this specification is intended to include all lower numerical limitations subsumed therein and any minimum numerical limitation recited in this specification is intended to include all higher numerical limitations subsumed therein. Accordingly, Applicant(s) reserves the right to amend this specification, including the claims, to expressly recite any sub-range subsumed within the ranges expressly recited herein. All such ranges are intended to be inherently described in this specification such that amending to expressly recite any such sub-ranges would comply with the requirements of 35 U.S.C. § 112 and 35 U.S.C. § 132(a).

The grammatical articles "one", "a", "an", and "the", as used in this specification, are intended to include "at least one" or "one or more", unless otherwise indicated. Thus, the articles are used in this specification to refer to one or more than one (i.e., to "at least one") of the grammatical objects of the article. By way of example, "a component" means one or more components, and thus, possibly, more than one component is contemplated and may be employed or used in an implementation of the described embodiments. Further, the use of a singular noun includes the plural, and the use of a plural noun includes the singular, unless the context of the usage requires otherwise.

As indicated, in certain embodiments, the present specification is directed to processes for removing alkali metal ions from polyether polyols. Such removal of alkali metal ions may sometimes be referred to herein as purifying the polyether polyol.

The polyether polyols subject to the processes of the present specification can be prepared in accordance with well-known procedures in which one or more alkylene oxides having from 2 to 10 carbon atoms, such as 2 to 6 carbon atoms, in the alkylene radical, and which are optionally substituted, are added to a starter molecule, which contains at least 2, such as 2 to 8, or, in some cases, 2 to 4 active hydrogen atoms, in the presence of an alkaline catalyst. The processes of the present specification are suitable for removing water and alkaline catalyst salts from a wide range of polyether polyols, in terms of their functionality, molecular weight and hydroxyl (OH) number.

Suitable alkylene oxides include, but are not limited to, butylene oxide, styrene oxide, ethylene oxide and propylene oxide. The alkylene oxides may be used individually, sequentially or as mixtures of two or more thereof.

Suitable starter molecules include, for example: aliphatic and aromatic N-mono-, N,N- and N,N'-dialkyl substituted diamines with 1 to 4 carbon atoms in the alkyl radical such as mono- and dialkyl substituted ethylenediamine, diethylenetriamine, triethylene-tetramine, 1,3-propylenediamine, 1,3- and/or 1,4-butylenediamine, 1,2-, 1,3-, 1,4-, 1,5- and 1,6-hexamethylenediamine, phenylenediamine, 2,4- and 2,6-toluenediamine, 4,4'-, 2,4- and 2,2'-diaminodiphenylmethane and mixtures of diaminodiphenylmethanes, etc.

Other suitable starter molecules include alkanolamines, such as ethanolamine, diethanolamine, N-methyl- and N-ethyl alkanolamines, such as N-methyl- and N-ethyl-diethanolamine and triethanolamine, ammonia, etc. In some embodiments, the starter molecules include monofunctional compounds such as, for example, butyl carbitol, and multifunctional, particularly bi- and/or trifunctional compounds such as, for example, water, ethylene glycol, 1,2-propylene glycol and trimethylene glycol, diethylene glycol, dipropylene glycol, 1,4-butanediol, 1,6-hexamethylene glycol, glycerine, trimethylol-propane, pentaerythritol, sorbitol and sucrose. The starter molecules may be used individually or as mixtures.

In certain embodiments, the processes of the present specification are particularly advantageous for use in connection with removing alkali metal ions from a polyether polyol that has a number average molecular weight of at least 150 gram/mole, such as at least 250 gram/mole, in some cases 700 gram/mole to 12,000 gram/mole, or, in some cases, 1000 to 12,000 gram/mole, and a hydroxyl number of 28 to 1050 mg KOH/gram, such as 28 to 650 mg KOH/gram, determined according to ASTM D6342-12. The number average molecular weights of the polyols described herein are calculated from the polyol's functionality and hydroxyl number according to the equation:

$$M_n = \frac{56100 * f}{OH\#}$$

in which $f$ is the functionality of the polyol (i.e., the number of hydroxyl groups per molecule), $OH\#$ is the hydroxyl number of the polyol and is equal to the mass in milligrams of potassium hydroxide (56.1 grams/mol) equivalent to the hydroxyl content in one gram of the polyol compound (mg KOH/g), and $M_n$ is the number average molecular weight of the polyol. The polyol functionality referred to herein is the theoretical average nominal functionality of the polyol, *i.e.,* the functionality calculated based on the average number of hydroxyl groups per molecule of starter used to produce the polyol.

An alkali metal ion-containing catalyst is used in the preparation of the polyether polyol that is subject to the processes of the present specification. Examples of such catalysts are alkali alkoxides with 1 to 4 carbon atoms in the alkyl radical, such as, but not limited to, sodium methylate, sodium and potassium ethylate, potassium isopropylate and sodium butylate, and alkali hydroxides, such as sodium hydroxide and potassium hydroxide. Such catalysts may be used individually or as mixtures of two or more thereof. In certain embodiments, the catalyst is present in an amount of 0.01 to 5 weight percent, 0.2 to 3 weight percent, or, in some cases, 0.1 to 1.0 weight percent, based on the total weight of polyether polyol present.

In embodiments of the processes of the present specification, the alkali-metal ion containing catalyst and polyether polyol are, prior to purification of the polyether polyol, present in a mixture that also includes water. In certain embodiments of the processes of the present specification, prior to any purification of the polyether polyol, water is present in such a mixture in an amount of at least 1% by weight, at least 3% by weight, or, in some cases, at least 4% by weight and up to 15% by weight, such as up to 13% by weight, up to 10% by weight, or up to 8% by weight, based on the total weight of polyether polyol present. In other embodiments, the alkali-metal ion containing catalyst and polyether polyol are, prior to purification of the polyether polyol, present in a mixture that is substantially free of water. As used herein, the term "substantially free" when used with reference to the substantial absence of water from the mixture comprising catalyst and polyether polyol, means that water is present in such a mixture in an amount of less than 1% by weight, such as less than 0.5% by weight, based on the total weight of polyether polyol present.

According to the processes of the present specification, a mixture comprising water and a polar organic solvent ("First Mixture") is combined with a mixture comprising a polyether polyol and an alkali metal ion-containing catalyst ("Second Mixture"). Suitable polar organic solvents include, for example, $C_1$ to $C_4$ alkyl alcohols, such as methanol, ethanol, propanol, isopropanol, butanol, and tert-butanol, including mixtures thereof. In certain embodiments, the First Mixture is added in an amount of 20 to 40% by weight, based on the total weight of the First Mixture and the Second Mixture. In some embodiments, the relative weight ratio of polar organic solvent and water in the First Mixture is within a range of 1:1 to 10:1, such as 2:1 to 8:1, or, in some cases 3:1 to 5:1.

In the processes of the present specification, the product of the foregoing combining step is passed through a bed comprising a cation exchange resin that is disposed in a container in order to remove alkali metal ion from the mixture. Suitable cation exchange resins include those of the gel type and those of the porous type and include, for example, resins having sulfonic acid groups ($-SO_3^-H^+$) and/or resins having carboxylic acid groups (-COOH), such as, for example, those based on crosslinked polystyrene, including, without limitation, copolymers of styrene and divinylbenzene with sulfonic acid groups and/or carboxylic acids groups, as well as (meth)acrylic acid functional polymers. As used herein, the term "(meth)acrylic" is meant to encompass methacrylic and acrylic. Suitable cation exchange resins are commercially available and include, for example, those under the tradenames Amberlite™ (Dow), Lewatit® (Lanxess), Dowex™ (Dow), Diaion™ (Mitsubishi Chemical), and Relite™ (Resindion), to name a few. In certain embodiments of the processes of the present specification, the bed comprising cation exchange resin has a volume of at least 10 cubic feet (0.28 cubic meter), such as at least 100 cubic feet (2.8 cubic meters), such as 10 to 1000 cubic feet (0.28 to 28 cubic meters), 100 to 1000 cubic feet (2.8 to 28 cubic meters), 100 to 500 cubic feet (2.8 to 14.2 cubic meters), or, in some cases, 200 to 400 cubic feet (5.7 to 11.3 cubic meters).

In some embodiments, the bed may also include an anion exchange resin and, as a result, in these embodiments, a so-called "mixed bed" is used. Suitable anion exchange resins include, without limitation, those that include quaternary ammonium groups, such as trimethylammonium groups, and/or amino groups, such as primary, secondary and/or tertiary amino groups. Suitable anion exchange resins include, without limitation, those based on crosslinked polystyrene.

In certain embodiments, the product of the foregoing combining step is passed through the bed of cation exchange resin at a resin bed temperature within the range of 40 to 150°C, such as 40 to 80°C, and/or at a container pressure of 50 to 120 pounds per square inch [absolute] (345 to 825 kilopascal), such as 65 to 85 pounds per square inch (445 to 590 kilopascal). In certain embodiments, at least 0.3%, such as at least 3.0%, of the alkali metal ions are removed from the polyether polyol containing mixture as it passes through the bed of cation exchange resin. In certain embodiments, the purified polyether polyol that exits the container has an alkali metal ion content of no more than 100 ppm, such as no more than 10 ppm, no more than 5 ppm, or, in some cases, no more than 1 ppm.

A critical feature of the processes of the present specification is that the container is operated liquid-full throughout the process. As used herein, when it is stated that the container is operated liquid-full throughout the processes of the present specification, it means that the liquid level in the container in which the cation exchange resin is disposed is maintained such that there is little or no gas/liquid interface in the container during the process and/or that the liquid level is maintained above the level of the bed of cation exchange resin throughout the process. In some embodiments, therefore, the liquid level is maintained at at least 90% of the total container height throughout the process, such as at at least 95% of the total container height, or, in yet other cases, at at least 99% of the total container height throughout the process. In some embodiments, the liquid level is maintained at 100% of the total container height throughout the process and, as such, in these embodiments of the process there is no gas/liquid interface in the container throughout the process. In some embodiments, no gas is added to the container to maintain a gas/liquid interface and/or the rate at which liquid is pumped out of the container is not controlled so as to maintain a gas/liquid interface in the container during the process. Rather, in some embodiments of the process, liquids are moved through the container by virtue of feed pressure to the container, such as pump pressure or other pressure sources, thereby eliminating, or at least virtually eliminating, back mixing of the polyether polyol. As used herein, "throughout the process" means that the container is maintained liquid-full continuously while the polyether polyol mixture is passed there through for the purpose of removing alkali metal ions therefrom.

In fact, it was discovered, surprisingly, that such liquid-full operation of the container in which the cation exchange resin is disposed in the processes of the present specification had a dramatically unexpected improvement on the alkali metal ion removal efficiency relative to a process utilizing liquid level control, which has led to a significant decrease in the frequency of cation exchange resin regenerations that need to be performed. In certain embodiments of the processes of the present specification, for example, at least 100, in some cases at least 110, kilograms of alkali metal ions are removed per cubic meter of resin present per regeneration in the processes of the present specification.

After passing through the container, the purified polyether polyol is often further processed to remove organic solvent and water. Such further processing often involves distillation, including distillation under at least atmospheric and/or vacuum conditions, either of which may be carried out batchwise or continuously. In some embodiments, for example, a combination of at least atmospheric distillation and vacuum distillation is used.

For example, in some embodiments, the at least atmospheric distillation step is used to remove a portion of the water and/or organic solvent under at least atmospheric pressure. As used herein, "atmospheric pressure" is synonymous with barometric pressure and refers to the pressure exerted by the weight of the atmosphere in the location in which the purified polyether polyol is disposed. In certain embodiments, for this at least atmospheric distillation, the temperature of the polyether polyol is maintained within the range of 100 to 130°C, such as 100 to 120°C. In certain embodiments, the at least atmospheric distillation is conducted at a pressure above atmospheric pressure, such as up to 30 psia (207 kPa).

In some embodiments, for example, an evaporator removes additional water and/or organic solvent from the polyether polyol under vacuum. Transition to the vacuum distillation step consists of reducing the pressure to the range of 50 mmHg to 5 mmHg, and, in some embodiments, to temperatures ranging from 100 to 140°C.

In certain embodiments, following distillation, water is present with the polyether polyol in an amount of no more than 10,000 ppm, no more than 1,000 ppm, or no more than 500 ppm (when measured according to ASTM D4672 (2012)).

As indicated, regeneration of the cation exchange resin is eventually necessary with the processes of the present invention. It is sometimes desirable to analytically measure the alkali metal-ion content in the effluent from the container to determine when regeneration is necessary. Regeneration of the cation exchange resin can be done by treating the resin with an acid, such as hydrochloric acid and/or sulfuric acid, though other mineral acids can be used. In some embodiments, an acid solution having acid concentration of 1 to 20% by weight, such as 2 to 10% by weight is used.

The polyether polyols purified by the processes of the present specification may be used in a variety of applications. For example, such polyether polyols may be reacted with one or more isocyanates to provide polyurethane products including, but not limited to, coatings, adhesives, sealants, elastomers, foams, including flexible foams, and the like. Suitable organic polyisocyanates for forming such polyurethanes include unmodified isocyanates, modified polyisocyanates, and isocyanate prepolymers. Such organic polyisocyanates include aliphatic, cycloaliphatic, araliphatic, aromatic, and heterocyclic polyisocyanates of the type described, for example, by W. Siefken in Justus Liebigs Annalen der Chemie, 562, pages 75 to 136. Examples of such isocyanates include those represented by the formula:

$$Q(NCO)_n$$

in which n is a number from 2-5, such as 2-3, and Q is an aliphatic hydrocarbon group; a cycloaliphatic hydrocarbon group; an araliphatic hydrocarbon group; or an aromatic hydrocarbon group.

Various aspects of the subject matter described in this specification are set out in the following embodiments:

Embodiment 1. A process for removing alkali metal ions from a polyether polyol, comprising: (a) combining a mixture of water and polar organic solvent with a mixture comprising a polyether polyol and an alkali metal ion-containing catalyst; and (b) passing the product of step (a) through a bed of a cation exchange resin that is disposed in a container to remove alkali metal ions therefrom, wherein the container is operated liquid-full throughout the process.

Embodiment 2. The process of Embodiment 1, wherein the polyether polyol has a calculated number average molecular weight 150 to 12,000 gram/mole.

Embodiment 3. The process of one or more of Embodiment 1 to Embodiment 2, wherein the polyether polyol has a hydroxyl number of 28 to 1050 mg KOH/gram determined according to ASTM D6342-12.

Embodiment 4. The process of one or more of Embodiment 1 to Embodiment 3, wherein the polar organic solvent comprises a $C_1$ to $C_4$ alkyl alcohol.

Embodiment 5. The process of one or more of Embodiment 1 to Embodiment 4, wherein the mixture of water and polar organic solvent is added in an amount of 20 to 40% by weight, based on the total weight of the mixture of water and polar organic solvent and the mixture comprising a polyether polyol and an alkali metal ion-containing catalyst.

Embodiment 6. The process of one or more of Embodiment 1 to Embodiment 5, wherein the relative weight ratio of polar organic solvent and water in the mixture of water and polar organic solvent is within a range of 1:1 to 10:1.

Embodiment 7. The process of Embodiment 6, wherein the relative weight ratio is 3:1 to 5:1.

Embodiment 8. The process of one or more of Embodiment 1 to Embodiment 7, wherein the cation exchange resin comprises carboxylic acid groups.

Embodiment 9. The process of one or more of Embodiment 1 to Embodiment 8, wherein the bed comprising cation exchange resin has a volume of at least 10 cubic feet.

Embodiment 10. The process of one or more of Embodiment 1 to Embodiment 9, wherein the bed comprising cation exchange resin has a volume of 100 to 1000 cubic feet.

Embodiment 11. The process of one or more of Embodiment 1 to Embodiment 10, wherein the product of step (a) is passed through the bed of cation exchange resin at a resin bed temperature within the range of range 40 to 80°C.

Embodiment 12. The process of one or more of Embodiment 1 to Embodiment 11, wherein the product of step (a) is passed through the bed of cation exchange resin at a container pressure of 65 to 85 pounds per square inch [absolute] (445 to 590 kilopascal).

Embodiment 13. The process of one or more of Embodiment 1 to Embodiment 12, wherein a purified polyether polyol that exits the container has an alkali metal ion content of no more than 100 ppm.

Embodiment 14. The process of Embodiment 13, wherein the purified polyether polyol that exits the container has an alkali metal ion content of no more than 1 ppm.

Embodiment 15. The process of one or more of Embodiment 1 to Embodiment 14, wherein the liquid level is maintained at at least 90% of the total container height throughout the process.

Embodiment 16. The process of one or more of Embodiment 1 to Embodiment 15, wherein the liquid level is maintained at at least 99% of the total container height throughout the process.

Embodiment 17. The process of one or more of Embodiment 1 to Embodiment 16, wherein there is no gas/liquid interface in the container throughout the process.

Embodiment 18. The process of one or more of Embodiment 1 to Embodiment 17, wherein liquids are moved through the container by virtue of feed pressure to the container so there is no back mixing of the polyether polyol.

Embodiment 19. The process of one or more of Embodiment 1 to Embodiment 18, wherein at least 100 kilograms of alkali metal ions are removed per cubic meter of resin present per regeneration.

Embodiment 20. The process of one or more of Embodiment 1 to Embodiment 19, further comprising removing organic solvent and water from the purified polyether polyol by distillation comprising at least atmospheric distillation wherein the temperature of the polyether polyol is maintained within the range of 100 to 130°C and the at least atmospheric distillation is conducted at a pressure above atmospheric pressure up to 30 psia (207 kPa).

Embodiment 21. The process of Embodiment 20, further comprising removing additional water and organic solvent from the polyether polyol under vacuum by reducing the pressure to the range of 50 mmHg to 5 mmHg at a temperature ranging from 100 to 140°C.

The non-limiting and non-exhaustive examples that follow are intended to further describe various non-limiting and non-exhaustive embodiments without restricting the scope of the embodiments described in this specification.

## EXAMPLES

Inventive Example: Polyether polyol containing 0.3 wt% KOH diluted with 30 wt% isopropanol and water (80 wt% isopropanol and 20% wt water) was fed into a resin bed operating under liquid full conditions, i.e., no gas/liquid interface in the container in which the resin bed was contained. The temperature of the bed was 60°C and the resin was Lewatit® CNP 80 WS resin (a weakly acidic, macroporous, acrylic-based cation exchange resin) from Lanxess. The flow continued until alkalinity of the polyether polyol was detected in the outlet polyether polyol stream of the resin bed indicating that the resin bed was exhausted. At this point the flow was stopped and the resin bed was regenerated. The amount of KOH removed before regeneration was necessary was tracked over a 2.5 year time period and determined to be 117 kg of potassium removed per cubic meter of resin present.

Comparative Example: Polyether polyol containing 0.3 wt% KOH diluted with 30 wt% isopropanol and water (80 wt% isopropanol and 20 wt% water) was fed into the same resin bed as in the Inventive Example with the exception that the bed operated under liquid level control wherein the liquid level was maintained just above the resin bed. The temperature was 60°C and the resin was Lewatit® CNP 80 WS resin from Lanxess. The flow continued until alkalinity of the polyether polyol was detected in the outlet polyether polyol stream of the resin bed indicating that the resin bed was exhausted. At this point the flow was stopped and the resin bed was regenerated. The amount of KOH removed before regeneration was necessary was tracked over a 2.5 year time period and determined to be 86 kg of potassium removed per cubic meter of resin present.

The examples demonstrate that liquid full operation of the resin bed provided an increased amount of KOH removal before the bed needs to be regenerated.

This specification has been written with reference to various non-limiting and non-exhaustive embodiments. However, it will be recognized by persons having ordinary skill in the art that various substitutions, modifications, or combinations of any of the disclosed embodiments (or portions thereof) may be made within the scope of this specification.

## Claims

1. A process for removing alkali metal ions from a polyether polyol, comprising:

   (a) combining a mixture of water and polar organic solvent with a mixture comprising a polyether polyol and an alkali metal ion-containing catalyst; and
   (b) passing the product of step (a) through a bed of a cation exchange resin that is disposed in a container to remove alkali metal ions therefrom,

   wherein the container is operated liquid-full throughout the process,
   and wherein the liquid-full operation of the container throughout the processes means that the liquid level in the container in which the cation exchange resin is disposed is maintained such that there is little or no gas/liquid interface in the container during the process and/or that the liquid level is maintained above the level of the bed of cation exchange resin throughout the process

2. The process of claim 1, wherein the polyether polyol has a calculated number average molecular weight 150 to 12,000 gram/mole as calculated using the equation given in the present specification.

3. The process of claim 1 or 2, wherein the polyether polyol has a hydroxyl number of 28 to 1050 mg KOH/gram determined according to ASTM D6342-12.

4. The process to one or more of claim 1 to 3, wherein the polar organic solvent comprises a $C_1$ to $C_4$ alkyl alcohol.

5. The process to one or more of claim 1 to 4, wherein the mixture of water and polar organic solvent is added in an amount of 20 to 40% by weight, based on the total weight of the mixture of water and polar organic solvent and the mixture comprising a polyether polyol and an alkali metal ion-containing catalyst.

6. The process to one or more of claim 1 to 5, wherein the relative weight ratio of polar organic solvent and water in the mixture of water and polar organic solvent is within a range of 1:1 to 10:1, preferably 3:1 to 5:1.

7. The process to one or more of claim 1 to 6, wherein the cation exchange resin comprises carboxylic acid groups.

8. The process to one or more of claim 1 to 7, wherein the bed comprising cation exchange resin has a volume of at least 0.28 $m^3$ (10 cubic feet), preferably 2.83 $m^3$ (100 cubic feet) to 28.32 $m^3$ (1000 cubic feet).

9. The process to one or more of claim 1 to 8, wherein the product of step (a) is passed through the bed of cation exchange resin at a resin bed temperature within the range of range 40 to 80°C.

10. The process to one or more of claim 1 to 9, wherein the product of step (a) is passed through the bed of cation exchange resin at a container pressure of 445 to 590 kilopascal (65 to 85 pounds per square inch (absolute)).

11. The process to one or more of claim 1 to 10, wherein a purified polyether polyol that exits the container has an alkali metal ion content of no more than 100 ppm, preferably of no more than 1 ppm.

12. The process one or more of claim 1 to 11, wherein the liquid level is maintained at at least 90% , preferably at at least 99% of the total container height throughout the process.

13. The process to one or more of claim 1 to 12, wherein liquids are moved through the container by virtue of feed pressure to the container so there is no back mixing of the polyether polyol.

14. The process to one or more of claim 1 to 13, wherein at least 100 kilograms of alkali metal ions are removed per cubic meter of resin present per regeneration.

15. The process to one of claim 1 to 14, further comprising removing organic solvent and water from the purified polyether polyol by distillation comprising at least atmospheric distillation wherein the temperature of the polyether polyol is maintained within the range of 100 to 130°C and the at least atmospheric distillation is conducted at a pressure above atmospheric pressure up to 207 kPa (30 psia) and further comprising removing additional water and organic solvent from the polyether polyol under vacuum by reducing the pressure to the range of 6666,1 Pa (50 mmHg) to 666,6 Pa (5 mmHg) at a temperature ranging from 100 to 140°C.

**Patentansprüche**

1. Verfahren zum Entfernen von Alkalimetallionen aus einem Polyetherpolyol, umfassend:

(a) Kombinieren eines Gemischs aus Wasser und polarem organischem Lösungsmittel mit einem Gemisch, das ein Polyetherpolyol und einen Alkalimetallionen enthaltenden Katalysator umfasst; und
(b) Leiten des Produkts von Schritt (a) durch ein Bett eines Kationenaustauschharzes, das in einem Behälter verteilt ist, um Alkalimetallionen daraus zu entfernen,

wobei der Behälter während des gesamten Verfahrens im vollständig mit Flüssigkeit gefüllten Zustand betrieben wird, und wobei der Betrieb des Behälters im vollständig mit Flüssigkeit gefüllten Zustand während des gesamten Verfahrens bedeutet, dass der Flüssigkeitsspiegel in dem Behälter, in dem das Kationenaustauschharz verteilt ist, derart gehalten wird, dass während des Verfahrens wenig oder keine Gas-/Flüssigkeits-Grenzfläche in dem Behälter vorhanden ist und/oder dass der Flüssigkeitsspiegel während des gesamten Verfahrens über dem Spiegel des Bettes des Kationenaustauschharzes gehalten wird.

2. Verfahren nach Anspruch 1, wobei das Polyetherpolyol ein berechnetes Molekulargewichtszahlenmittel von 150 bis 12.000 g/mol, wie es mit der in der vorliegenden Beschreibung angegebenen Gleichung berechnet wurde, aufweist.

3. Verfahren nach Anspruch 1 oder 2, wobei das Polyetherpolyol eine Hydroxylzahl von 28 bis 1050 mg KOH/g, bestimmt gemäß ASTM D6342-12, aufweist.

4. Verfahren nach einem oder mehreren von Anspruch 1 bis 3, wobei das polare organische Lösungsmittel einen $C_1$- bis $C_4$-Alkylalkohol umfasst.

5. Verfahren nach einem oder mehreren von Anspruch 1 bis 4, wobei das Gemisch aus Wasser und polarem organischem Lösungsmittel in einer Menge von 20 bis 40 Gew.-% zugesetzt wird, bezogen auf das Gesamtgewicht des Gemischs aus Wasser und polarem organischem Lösungsmittel und dem Gemisch, umfassend ein Polyetherpolyol und einen Alkalimetallionen enthaltenden Katalysator.

6. Verfahren nach einem oder mehreren von Anspruch 1 bis 5, wobei das relative Gewichtsverhältnis von polarem organischem Lösungsmittel und Wasser in dem Gemisch von Wasser und polarem organischem Lösungsmittel in einem Bereich von 1:1 bis 10:1, vorzugsweise 3:1 bis 5:1, liegt.

7. Verfahren nach einem oder mehreren von Anspruch 1 bis 6, wobei das Kationenaustauschharz Carbonsäuregruppen umfasst.

8. Verfahren nach einem oder mehreren von Anspruch 1 bis 7, wobei das Bett, das ein Kationenaustauschharz umfasst, ein Volumen von mindestens 0,28 m$^3$ (10 Kubikfuß), vorzugsweise 2,83 m$^3$ (100 Kubikfuß) bis 28,32 m$^3$ (1000 Kubikfuß) aufweist.

9. Verfahren nach einem oder mehreren von Anspruch 1 bis 8, wobei das Produkt von Schritt (a) bei einer Harzbetttemperatur im Bereich von 40 bis 80°C durch das Bett aus Kationenaustauschharz geleitet wird.

10. Verfahren nach einem oder mehreren von Anspruch 1 bis 9, wobei das Produkt von Schritt (a) bei einem Behälterdruck von 445 bis 590 Kilopascal (65 bis 85 Pfund pro Quadratzoll (absolut)) durch das Bett aus Kationenaustauschharz geleitet wird.

11. Verfahren nach einem oder mehreren von Anspruch 1 bis 10, wobei ein gereinigtes Polyetherpolyol, das aus dem Behälter austritt, einen Alkalimetallionengehalt von nicht mehr als 100 ppm, vorzugsweise nicht mehr als 1 ppm, aufweist.

12. Verfahren nach einem oder mehreren von Anspruch 1 bis 11, wobei der Flüssigkeitsspiegel während des gesamten Verfahrens bei mindestens 90%, vorzugsweise mindestens 99% der gesamten Behälterhöhe gehalten wird.

13. Verfahren nach einem oder mehreren von Anspruch 1 bis 12, wobei Flüssigkeiten aufgrund des Förderdrucks zu dem Behälter durch den Behälter bewegt werden, so dass kein Rückmischen des Polyetherpolyols erfolgt.

14. Verfahren nach einem oder mehreren von Anspruch 1 bis 13, wobei mindestens 100 kg Alkalimetallionen pro Kubikmeter Harz, die pro Regeneration vorhanden sind, entfernt werden.

15. Verfahren nach einem von Anspruch 1 bis 14, zudem umfassend das Entfernen von organischem Lösungsmittel und Wasser aus dem gereinigten Polyetherpolyol durch Destillation, umfassend mindestens atmosphärische Destillation, wobei die Temperatur des Polyetherpolyols im Bereich von 100 bis 130°C gehalten wird und die mindestens atmosphärische Destillation bei einem Druck über dem Atmosphärendruck bis zu 207 kPa (30 psia) durchgeführt wird, und zudem umfassend das Entfernen von zusätzlichem Wasser und organischem Lösungsmittel aus dem Polyetherpolyol unter Vakuum durch Reduzieren des Drucks auf den Bereich von 6666,1 Pa (50 mmHg) bis 666,6 Pa (5 mmHg) bei einer Temperatur im Bereich von 100 bis 140°C.

**Revendications**

1. Procédé pour l'élimination d'ions de métaux alcalins d'un polyol de polyéther, comprenant :

   (a) la combinaison d'un mélange d'eau et de solvant organique polaire avec un mélange comprenant un polyol de polyéther et un catalyseur contenant des ions de métaux alcalins ; et
   (b) le passage du produit de l'étape (a) dans un lit d'une résine échangeuse de cations qui est disposée dans un récipient pour éliminer des ions de métaux alcalins de celui-ci,

   dans lequel le récipient est amené à fonctionner rempli de liquide pendant tout le procédé et dans lequel le fonctionnement rempli de liquide du récipient dans tout le procédé signifie que le niveau de liquide dans le récipient dans lequel la résine échangeuse de cations est disposée est maintenu de façon telle qu'il y a peu ou pas d'interface gaz/liquide dans le récipient pendant le procédé et/ou que le niveau de liquide est maintenu au-dessus du niveau du lit de résine échangeuse de cations pendant tout le procédé.

2. Procédé selon la revendication 1, dans lequel le polyol de polyéther a une masse moléculaire moyenne en nombre calculée telle que calculée à l'aide de l'équation donnée dans la présente description de 150 à 12 000 grammes/mole.

3. Procédé selon la revendication 1 ou 2, dans lequel le polyol de polyéther a un indice d'hydroxyle déterminé selon la norme ASTM D6342-12 de 28 à 1050 mg de KOH/gramme.

4. Procédé selon une ou plusieurs des revendications 1 à 3, dans lequel le solvant organique polaire comprend un alcool alkylique en $C_1$ à $C_4$.

5. Procédé selon une ou plusieurs des revendications 1 à 4, dans lequel le mélange d'eau et de solvant organique polaire est ajouté en une quantité de 20 à 40 % en poids, par rapport au poids total du mélange d'eau et de solvant organique polaire et du mélange comprenant un polyol de polyéther et un catalyseur contenant des ions de métaux alcalins.

6. Procédé selon une ou plusieurs des revendications 1 à 5, dans lequel le rapport pondéral relatif de solvant organique polaire et d'eau dans le mélange d'eau et de solvant organique polaire est dans une plage de 1:1 à 10:1, de préférence de 3:1 à 5:1.

7. Procédé selon une ou plusieurs des revendications 1 à 6, dans lequel la résine échangeuse de cations comprend des groupes acide carboxylique.

8. Procédé selon une ou plusieurs des revendications 1 à 7, dans lequel le lit comprenant de la résine échangeuse de cations a un volume d'au moins 0,28 m$^3$ (10 pieds cubes), de préférence de 2,83 m$^3$ (100 pieds cubes) à 28,32 m$^3$ (1000 pieds cubes).

9. Procédé selon une ou plusieurs des revendications 1 à 8, dans lequel le produit de l'étape (a) est amené à passer dans le lit de résine échangeuse de cations à une température de lit de résine dans la plage de 40 à 80 °C.

10. Procédé selon une ou plusieurs des revendications 1 à 9, dans lequel le produit de l'étape (a) est amené à passer dans le lit de résine échangeuse de cations à une pression de récipient de 445 à 590 kilopascals (65 à 85 livres par pouce carré (pression absolue)).

11. Procédé selon une ou plusieurs des revendications 1 à 10, dans lequel un polyol de polyéther purifié qui sort du récipient a une teneur en ions de métaux alcalins de pas plus de 100 ppm, de préférence de pas plus de 1 ppm.

12. Procédé selon une ou plusieurs des revendications 1 à 11, dans lequel le niveau de liquide est maintenu à au moins 90 %, de préférence à au moins 99 % de la hauteur de récipient totale pendant tout le procédé.

13. Procédé selon une ou plusieurs des revendications 1 à 12, dans lequel des liquides sont amenés à se déplacer dans le récipient en vertu de la pression d'alimentation appliquée au récipient de sorte qu'il n'y a pas de mélange à contre-courant du polyol de polyéther.

14. Procédé selon une ou plusieurs des revendications 1 à 13, dans lequel au moins 100 kilogrammes d'ions de métaux alcalins sont éliminés par mètre cube de résine présente par régénération.

15. Procédé selon l'une des revendications 1 à 14, comprenant en outre l'élimination de solvant organique et d'eau du polyol de polyéther purifié par distillation comprenant une distillation au moins atmosphérique dans lequel la température du polyol de polyéther est maintenue dans la plage de 100 à 130 °C et la distillation au moins atmosphérique est mise en œuvre à une pression au-dessus de la pression atmosphérique allant jusqu'à 207 kPa (30 lb/po$^2$ abs.) et comprenant en outre l'élimination d'eau et de solvant organique supplémentaires du polyol de polyéther sous vide par réduction de la pression à la plage de 6666,1 Pa (50 mm de Hg) à 666,6 Pa (5 mm de Hg) à une température allant de 100 à 140 °C.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030150814 A1
- EP 0376157 A2
- US 4994627 A

**Non-patent literature cited in the description**

- **W. SIEFKEN.** *Justus Liebigs Annalen der Chemie,* vol. 562, 75-136